# EUROPEAN PATENT APPLICATION

(11) **EP 4 306 141 A1**
(43) Date of publication of application: **17.01.2024**
(21) Application number: 22767533.7
(22) Date of filing: 10.03.2022
(51) Int. Cl.: A61L 33/00, A61L 29/08, A61M 25/00

(54) **METHOD FOR GRAFTING SURFACE OF MEDICAL DEVICE BY USING QUATERNIZED TRIMETHYLAMINE**

(30) Priority: 11.03.2021 KR 20210031883; 08.03.2022 KR 20220029513
(71) Applicant: Vasular Interface Inc., Suwon-si, Gyeonggi-do 16426 (KR)
(72) Inventor: KIM, Do Yeon, Gwangju-si Gyeonggi-do 12789 (KR); KANG, Jong Hee, Yongin-si Gyeonggi-do 17056 (KR); HAN, Su Gyung, Hwaseong-si Gyeonggi-do 18385 (KR)
(74) Representative: Grünecker Patent- und Rechtsanwälte PartG mbB
(86) International application number: PCT/KR2022/003363
(87) International publication number: WO 2022/191637

(57) **Abstract**

The present invention relates to a method for grafting a surface of a medical device by using quaternized trimethylamine. A catheter onto which quaternized trimethylamine has been grafted allows for the reduction of thrombus production. Therefore, the catheter having quaternized trimethylamine grafted thereto exhibits the effect of repressing thrombus production and enhancing the function of inhibiting blood adsorption and blood cell adsorption.

## Description

### [Technical Field]

The present invention relates to a method for grafting the surface of medical devices with quaternized trimethylamine.

### [Background Art]

Methods of coating conventional various medical devices including cardiovascular systems that come into contact with blood with antibacterial and antithrombotic materials have been developed to inhibit infection and clot formation, but the problems such as weak safety of surface coating, difficulty in the coating process and toxicity in the body have been raised and remain completely unsolved. Therefore, there is a highly increasing need to develop a high versatile and antithrombotic and antibacterial polymer and a chemical additive composition containing the same.

In general, protein adsorption occurs spontaneously on the surface of a medical material in contact with blood. As a result, cells and various ingredients in the blood are slowly dispersed on and attached to the surface of the medical material on which proteins are adsorbed. Protein adsorption not only reduces the functions of medical materials, but also causes side effects such as blood clotting and inflammation. In addition, protein adsorption reduces the sensitivity of medical device sensors inserted to determine the health conditions of patients, thus reducing diagnostic efficiency. Therefore, strategies to suppress protein adsorption, which is a primary phenomenon occurring when inserted into the body, may be very effective in blood contact-type medical devices inserted into the body, from the viewpoint of research and development.

In general, blood clots are formed by adsorption of proteins and platelets in the blood. When blood clots are formed, blood circulation is not smooth, causing various diseases in the human body. In particular, upper deep vein thrombosis caused by medical devices is a very serious disease that requires immediate treatment. The formation of blood clots may also lead to infection of the bloodstream. Among nosocomial infections, bloodstream infections are the second most common infection after urinary tract infection. This bloodstream infection commonly occurs in catheters among blood contact-type medical devices and accounts for about 30% or more of all nosocomial infection cases.

### [Disclosure]

### [Technical Problem]

Unnecessary social costs and patient costs are required to prevent various diseases caused by protein adsorption, such as formation of blood clots and infection by blood clots. For this reason, there is a need for functional medical devices that inhibit protein adsorption, blood clot formation, and biofilm formation.

Among medical devices, blood-contacting medical devices generally have two problems. The first is clot formation caused by protein adsorption and the second is bloodstream infection by blood clotting. Methods for improving blood compatibility and inhibiting protein adsorption and blood clotting by modifying the surface of medical devices may be used in order to solve these side effects.

### [Technical Solution]

As a result of performing a graft-from method using quaternized trimethylamine, the present inventors found that blood contact-type medical devices exhibiting excellent functions of inhibiting blood adsorption and blood cell adsorption, and having a small inner diameter from 10 gauge to 20 gauge can be used as medical devices inserted into the human body without a separate solute and solvent circulation device.

In accordance with one aspect of the present invention, the above and other objects can be accomplished by the provision of a method of grafting quaternized trimethylamine on the surface of a substrate, including mixing a reaction solvent, a catalyst, an initiator, and a compound having an isocyanate group with a substrate, followed by stirring, to modify the surface of the substrate, further mixing the surface-modified substrate with a reducing agent and a trimethylamine-based compound, followed by reacting, to form a trimethylamine polymer layer on the surface of the substrate, and further mixing the substrate with hydrogen peroxide or meta-chloroperoxybenzoic acid, followed by reacting, to form a quaternized trimethylamine graft polymer layer on the surface of the substrate.

In accordance with another aspect of the present invention, provided is a method of grafting quaternized trimethylamine on the surface of a substrate, including introducing a hydroxyl group (-OH) into the surface of the substrate, further mixing the hydroxyl group-introduced substrate with an initiator, a reducing agent, and a trimethylamine-based compound, followed by reacting, to form a trimethylamine polymer layer on the surface of the substrate, and further mixing the substrate with hydrogen peroxide or meta-chloroperoxybenzoic acid, followed by reacting, to form a quaternized trimethylamine graft polymer layer on the surface of the substrate.

In accordance with another aspect of the present invention, provided is a method of grafting quaternized trimethylamine on the surface of a substrate, including mixing a reaction solvent and an initiator with a substrate, followed by stirring, to modify the surface of the substrate, further mixing the surface-modified substrate with a reducing agent and a trimethylamine-based compound, followed by reacting, to form a trimethylamine polymer layer on the surface of the substrate, and further mixing the substrate with hydrogen peroxide or meta-chloroperoxybenzoic acid, followed by reacting, to form a quaternized trimethylamine graft polymer layer on the surface of the substrate.

### [Advantageous effects]

The present invention relates to a method for grafting the surface of a medical device with quaternized trimethylamine. The catheter used for grafting with quaternized trimethylamine has the effects of improving functions to inhibit blood clot formation, blood adsorption and blood cell adsorption.

### [Description of Drawings]

FIG. 1 is a schematic diagram illustrating an implantable medical device including a graft polymer layer according to an embodiment.
FIG. 2 is a schematic diagram illustrating a method of grafting an implantable medical device according to Example 1.
FIG. 3 is a schematic view illustrating a method of grafting an implantable medical device according to Example 2.
FIG. 4 is a schematic diagram illustrating a method of grafting an implantable medical device according to Example 3.
FIG. 5 is a schematic diagram illustrating a method of grafting an implantable medical device according to Example 4;
FIG. 6 is a graph showing the results of infrared spectroscopy (FT-IR) of the substrate surface according to Example and Control group.
FIG. 7 is a graph showing the results of ¹H-NMR analysis of the substrate surface according to Comparative Example.
FIG. 8 shows the results of measurement of the contact angle of the surface of the substrate, on which the graft polymer layer is formed, according to Examples, Comparative Example and Control group.
FIG. 9 is an image showing the adsorption amount of fibrinogen in Examples, Comparative Example and Control group.

### [Best Mode]

Hereinafter, the present invention will be described in detail.

### Method for grafting quaternized trimethylamine on surface of substrate

In another aspect, the present invention is directed to a method of grafting quaternized trimethylamine on the surface of a substrate, including mixing a reaction solvent, a catalyst, an initiator, and a compound having an isocyanate group with a substrate, followed by stirring, to modify the surface of the substrate, further mixing the surface-modified substrate with a reducing agent and a trimethylamine-based compound, followed by reacting, to form a trimethylamine polymer layer on the surface of the substrate, and further mixing the substrate with hydrogen peroxide or meta-chloroperoxybenzoic acid, followed by reacting, to form a quaternized trimethylamine graft polymer layer on the surface of the substrate.

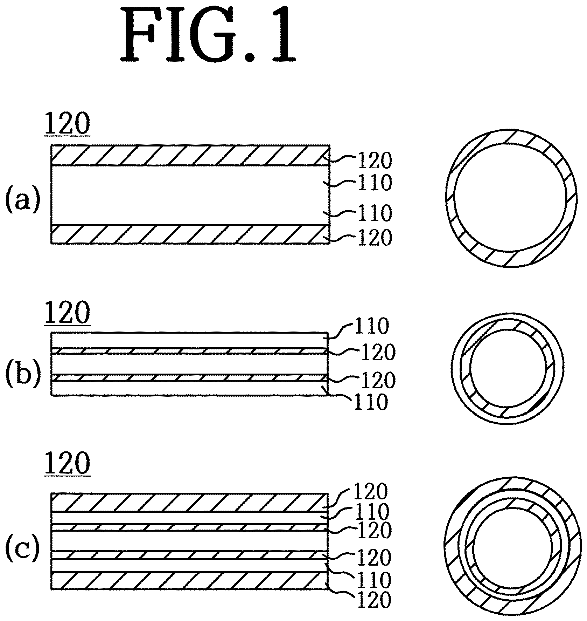

FIG. 1 is a schematic diagram illustrating an implantable medical device 100 (injectable medical device, or a medical device that is introduced into the body) including a graft polymer layer according to an embodiment.

Referring to FIG. 1, the implantable medical device 100 includes an implantable substrate 110 and a graft polymer layer 120, wherein the graft polymer layer 120 contains a quaternized trimethylamine graft copolymer.

In the present invention, the water contact angle of the surface of the substrate modified with quaternized trimethylamine may be 60° or less, and the amount of fibrinogen adsorbed thereon may be 1 µg/cm² or less. Specifically, the water contact angle is 20° or more and 50° or less, 20° or more and 40° or less, 20° or more and 30° or less, and the amount of fibrinogen adsorbed thereon is 0.03 µg/cm² or more and 0.5 µg/cm² or less, 0.03 µg/cm² or more and 0.3 µg/cm² or less, 0.03 µg/cm² or more and 0.1 µg/cm² or less, or 0.03 µg/cm² or more and 0.05 µg/cm² or less, but is not limited thereto.

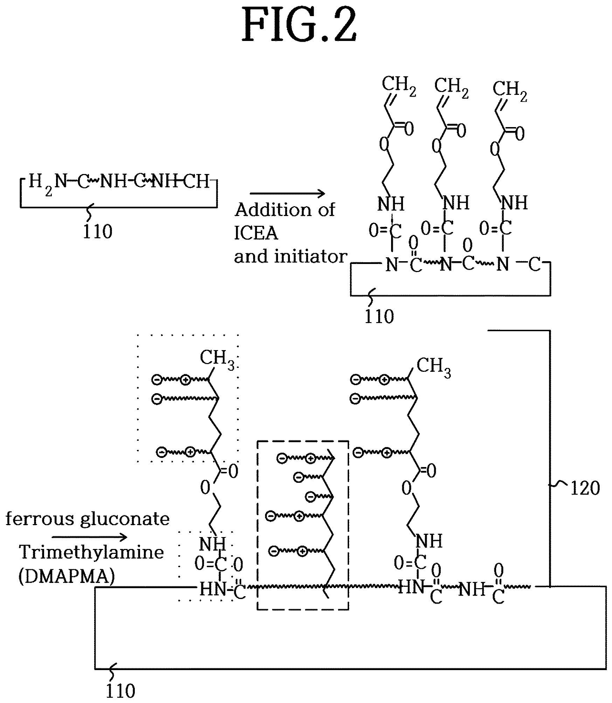

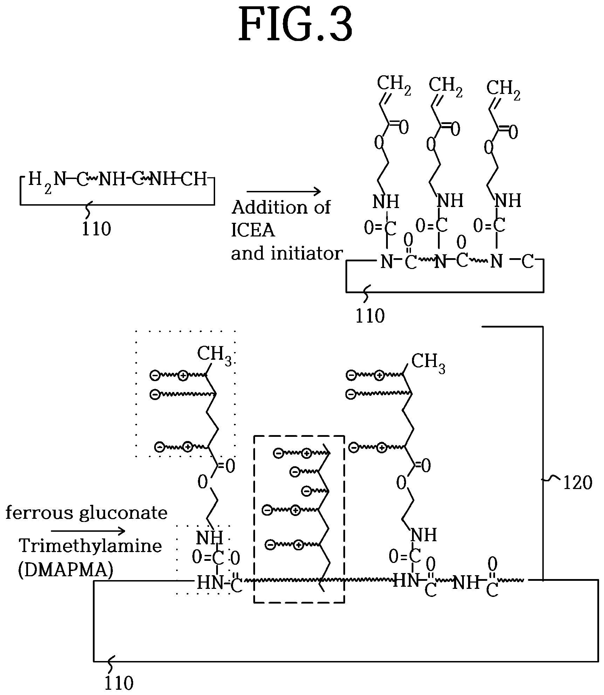

FIGS. 2 and 3 are schematic diagrams illustrating a method of grafting an implantable medical device according to one embodiment.

In detail, FIGS. 2 and 3 show a grafting method using polyurethane.

Referring to FIGS. 2 and 3, first, polyurethane 110, an initiator, 2-isocyanatoethyl acrylate (ICEA), and a dibutyltin dilaurate (FIG. 2)/bismuth catalyst (FIG. 3) are reacted in the presence of a reaction solvent to perform first surface modification.

As the first surface modification is performed, the polyurethane 110 is swollen by the reaction solvent and the initiator penetrates into the swollen polyurethane 110.

In addition, the -NH group of the polyurethane 110 reacts with the 2-isocyanatoethyl acrylate (ICEA) group to form a -C=C group on the surface of the polyurethane 110. At this time, the -NCO group of the 2-isocyanatoethyl acrylate (ICEA) is bonded to -NH on the surface of the polyurethane 110 and thus the -NCO group is chemically bonded to the polyurethane 110.

In the subsequent step (step 2), iron (II) gluconate (ferrous gluconate) and a trimethylamine-based compound are added to the surface-modified polyurethane 110 to perform second surface modification to thereby form a trimethylamine polymer layer on the surface of the polyurethane 110.

Specifically, as the second surface modification is performed, the initiator penetrating into the polyurethane 110 is initiated by the reducing agent to form radicals, and the formed radicals and the trimethylamine-based compound are polymerized to form a trimethylamine polymer layer.

That is, as the trimethylamine polymer layer polymerizes with radicals formed in the polyurethane 110 and thus forms a strong physical bond with the polyurethane 110.

As a last step (third step), hydrogen peroxide or meta-chloroperoxybenzoic acid is added to the polyurethane 110, on which the trimethylamine polymer layer is formed, and then mixed to react the trimethylamine-based compound with hydrogen peroxide or meta-chloroperoxybenzoic acid, and perform third surface modification to form the quaternized trimethylamine graft polymer layer 120 on the surface of the polyurethane 110.

The following Reaction Scheme 1 shows the chemical reaction mechanism of the third surface modification in FIGS. 2 and 3. Hydrogen peroxide (H₂O₂) or meta-chloroperoxybenzoic acid (MCPBA) reacts with the tertiary nitrogen moiety of dimethylamino propyl methacrylamide and dimethylamino propyl acrylamide, to induce oxidation to thereby form a quaternized trimethylamine grafted polymer layer. 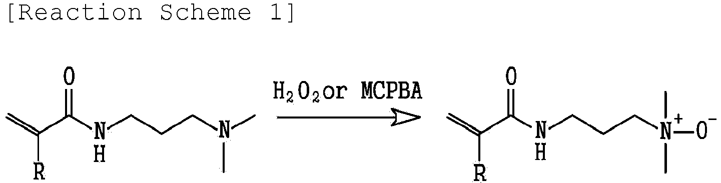 (In Reaction Scheme 1, R is hydrogen (H) or a methyl group (CH₃).)

Meanwhile, through the first surface modification, the C=C group formed on the surface of the polyurethane 110 and the trimethylamine-based compound are polymerized to form a trimethylamine polymer layer. At this time, a chemical bond is formed between the C=C group and the trimethylamine polymer layer.

That is, the trimethylamine polymer layer forms a strong physical bond in the polyurethane 110 and forms a chemical bond with the surface of the polyurethane 110, so that a trimethylamine polymer layer is formed as a polymer brush.

In the present invention, the method of grafting quaternized trimethylamine on the surface of the substrate includes mixing a reaction solvent, a catalyst, an initiator, a compound having an isocyanate group, with a substrate, followed by stirring, as a first step.

In the present invention, the reaction solvent is selected from the group consisting of distilled water, toluene, methanol (MeOH), ethanol (EtOH), dimethylformamide (DMF), acetone, ethyl acetate (EtOAc), acetonitrile (ACN), hexane, heptane, and isopropyl alcohol.

In the present invention, the catalyst may be selected from the group consisting of dibutyltin dilaurate, triethylamine, diethylenetriamine, bismuth carboxylate and zirconium chelate.

In the present invention, the initiator may react with an oxidizing agent or a reducing agent to form a radical. As a result, radical polymerization may be induced. In this case, the initiator may be a peroxide initiator. The radical polymerization is a polymerization method of forming polymers by continuously adding free radicals, and the radicals may be generally formed through various mechanisms using separate initiator molecules. Once the reaction is initiated, the formed radical forms a stable single bond using one electron of the pi bond from the polymer monomer containing the double bond, and the double bond is converted into a single bond to form new radicals containing an excess electron on other carbon atoms not bonded with the radical. A polymer chain may be grown through the newly formed radicals while repeating the process. The initiator may be introduced into the surface of the substrate by chemical adsorption and the initiator may include a reactive group that chemically reacts with the surface of the substrate to form a chemical bond between the substrate and the initiator.

In the present invention, the initiator may also be introduced into and/or on the surface of the substrate by physical adsorption, and the initiator is dissolved in a solvent or a combination thereof. The substrate is immersed in the solvent or solvent combination containing the initiator for a predetermined period of time, to physically adsorb the initiator on the surface thereof.

In the present invention, the initiator may be selected from the group consisting of an ultraviolet initiator, a thermal initiator and a redox initiator.

Specifically, the UV initiator may be 1-hydroxycyclohexyl phenyl ketone, 2,2-diethoxyacetophenone, 2-benzyl-2-(dimethylamino)-4'-morpholinobutyrophenone, 2-hydroxy-2-methylpropiophenone, 2-hydroxy-4'-(2-hydroxyethoxy)-2-methylpropiophenone, 2-methyl-4'-(methylthio)-2-methylpropiophenone, 3'-hydroxyacetophenone, 4'-ethoxyacetophenone, 4'-hydroxyacetophenone, 4'-phenoxyacetophenone, 4'-tert-butyl-2',6'-di methylacetophenone, diphenyl(2,4,6-trimethylbenzoyl)phosphine oxide/2-hydroxy-2-methylpropiophenone, 2,2-dimethoxy-2-phenylacetophenone, 4,4'-dimethoxybenzoin, 4,4'-dimethylbenzyl, benzoin ethyl ether, benzoin isobutyl ether, benzoin methyl ether, benzoin, 2-methylbenzophenone, 3,4-dimethylbenzophenone, 3-hydroxybenzophenone, 3-methylbenzophenone, 4,4'-bis(diethylamino)benzophenone, 4,4'-dihydroxybenzophenone, 4,4'-bis[2-(1)-propenyl)phenoxy]benzophenone, 4-(diethylamino)benzophenone, 4-benzoylbiphenyl, 4-hydroxybenzophenone, 4-methylbenzophenone, benzophenone-3,3',4,4'-tetracarboxylic dianhydride, benzophenone, methyl benzoylformate, Michler's ketone, sulfonium, iodium, 2-(4-methoxystyryl)-4,6-bis(trichloromethyl)-1,3,5-triazine, diphenyliodonium p-toluenesulfonate, N-hydroxy-5-norbornene-2,3-dicarboximide perfluoro-1-butanesulfonate, N-hydroxynaphthalimide triflate, 2-tert-butylanthraquinone, 9,10-phenanthrenequinone, anthraquinone-2-sulfonic acid sodium salt monohydrate, camphorquinone, diphenyl(2,4,6-trimethylbenzoyl)phosphine oxide, 10-methylphenothiazine, thioxanthone, and IRGACURE 2959, but is not limited thereto.

In addition, the thermal initiator is tert-amyl peroxybenzoate, 4,4-azobis(4-cyanovaleric acid), 2,2'-azobis[(2-carboxyethyl)-2-methylpropionamidine], 2,2'-azobis(4-methoxy-2,3,-dimethylvaleronitrile), 1,1'-azobis(cyclohexanecarbonitrile), 2,2'-azobisisobutyronitrile (AIBN), benzoyl peroxide, 2,2-bis(tert-butylperoxy)butane, 1,1-bis(tert-butylperoxy)cyclohexane, 2,5-bis(tert-butylperoxy)-2,5-dimethylhexane, 2,5-bis(tert-butylperoxy)-2,5-dimethyl-3-hexyne, bis(1-(tert-butylperoxy)-1-methylethyl)benzene, 1,1-bis(tert-butylperoxy)-3,3,5-trimethylcyclohexane, tert-butyl hydroperoxide, tert-butyl peracetate, tert-butyl peroxide, tert-butyl peroxybenzoate, tert-butylperoxy isopropyl carbonate, cumene hydroperoxide, cyclohexanone peroxide, dicumyl peroxide, lauroyl peroxide, 2,4-pentanedione peroxide, peracetic acid, and potassium sulfate, but is not limited thereto.

In addition, the redox initiator is used to initiate polymerization from the surface of the substrate, and the redox initiator typically includes a pair of initiators, that is, an oxidizing agent and a reducing agent. Redox initiation is regarded as a one-electron transfer reaction to efficiently generate free radicals under mild conditions. Suitable oxidizing agents include peroxides, hydroperoxides, persulfates, peroxycarbonates, peroxydisulfates, peroxydiphosphates, permanganates, metals such as Mn(III), Ce(IV), V(V), Co(III), Cr(VI) and Fe(III) salts, but are not limited thereto.

In addition, suitable reducing agents include metal salts such as Fe(II), Cr(II), V(II), Ti(III), Cu(II) and Ag(I) salts, and sulfur oxyacids, hydroxy acids, alcohols, thiols, ketones, aldehydes, amines and amides, but are not limited thereto. For example, in some embodiments, the reducing agent is an iron(II) salt, such as iron (II) L-ascorbate, ferrous sulfate, iron (II) acetate, iron (II) acetylacetonate, iron (II) ethylenediammonium sulfate, iron (II) gluconate, iron (II) lactate, iron (II) oxalate, or iron (II) sulfate.

In the present invention, the initiator may be selected from the group consisting of 2,2-bis(4,4-di(t-butylperoxy)cyclohexyl) propane, tri(t-butylperoxy) triazine, tri(t-butylperoxy)trimellitate, polyether poly-t-butyl peroxycarbonate, t-butyl peroxy methyl fumarate, and t-butyl peroxy ethyl fumarate. In an embodiment of the present invention, polyether poly-t-butylperoxy carbonate (product name: JWEB50) was used as an initiator.

In the present invention, the compound having an isocyanate group is selected from the group consisting of 2-isocyanatoethyl methacrylate (IEM), 2-isocyanatoethyl acrylate (ICEA), isophorone diisocyanate and hexamethylene diisocyanate. In one embodiment of the present invention, 2-isocyanatoethyl methacrylate (IEM) and 2-isocyanatoethyl methacrylate (ICEA) were used as the compound having an isocyanate group.

In the present invention, the compound having an isocyanate group is chemically bonded to the surface of the substrate through the -NCO group.

In the present invention, the method of grafting quaternized trimethylamine on the surface of the substrate includes further mixing the surface-modified substrate with a reducing agent and a trimethylamine-based compound, followed by reacting, to form a trimethylamine polymer layer on the surface of the substrate, as a second step.

In the present invention, the trimethylamine compound includes at least one selected from the group consisting of dimethylamino propyl methacrylamide, dimethylamino propyl acrylamide, and dimethylamino ethyl methacrylate, but is not limited thereto.

In the present invention, the trimethylamine-based compound is grafted onto the surface of a substrate such as polyurethane using the initiator bonded to the substrate surface in the first step, and the reducing agent and the trimethylamine-based compound added in the second step.

In the present invention, the reducing agent may be selected from the group consisting of metal salts, sulfur oxyacids, alcohols, hydroxy acids, thiols, ketones, amine aldehydes and amides.

In the present invention, the metal salt may be a salt of Fe(II), Cu(II), Cr(II), V(II), Ti(III) or Ag(I). In one embodiment of the present invention, a metal salt, specifically, ferrous gluconate was used as a reducing agent.

In the present invention, the method of grafting quaternized trimethylamine on the surface of the substrate includes further mixing the substrate with hydrogen peroxide or meta-chloroperoxybenzoic acid, followed by reacting, as a third step. The reaction for forming quaternized trimethylamine has been depicted in Reaction Scheme 1 above and thus is not described again.

In the present invention, the quaternized trimethylamine may be any one of quaternized dimethylamino propyl methacrylamide, quaternized dimethylamino propyl acrylamide and quaternized dimethylamino ethyl methacrylate.

In the present invention, the polymer film formed by the grafting of the present invention is formed from several nanometers below the surface of the medical device and is not damaged even by strong stimulation such as ultrasonication. Even in the implantable medical device grafted with the polymer, the adhesion of proteins and/or cells may be reduced and thus the risk of blood clot formation due to protein adsorption and infection due to clot formation may be greatly lowered. Among the surface modification methods other than the grafting method, the coating method easily causes peeling and thus is not suitable as surface modification for human circulatory devices because safety is not secured. Therefore, there is a need for a surface modification method that does not cause peeling even when stimulated externally or inside the human body. In addition, blood contact-type medical devices having a small inner diameter are clogged due to the stagnation of solutes and solvents in the interior thereof, making it difficult to perform a smooth function as a medical device. In order to solve this problem, quaternized trimethylamine is grafted onto implantable medical devices to secure both safety and effectiveness.

As used herein, the term "implantable medical device" is also simply referred to as "medical device" and encompasses blood contact-type medical devices wherein blood clotting often occur, medical devices with a high infection risk, and medical devices that are often infected with bacterial biofilms, and examples thereof include, but are not limited to, surgical instruments, medical or dental devices, respirators, drug delivery devices, scaffolds, valves, pacemakers, stents, catheters, rods, implants, fracture fixation devices, pumps, tubes, wires, electrodes, endoscopes, especially, other devices that come into contact with bio-tissues.

In the present invention, the substrate of the medical device may include a metal, ceramic, glass, a synthetic polymer, biological tissue, woven fiber, non-woven fabric, a semi-metal, or silicone.

In the present invention, the ceramic may be selected from the group consisting of oxides, carbides, and nitrides of transition metal elements or metalloid elements.

In the present invention, the metal may be selected from the group consisting of titanium and alloys thereof, stainless steel, tantalum, palladium, zirconium, niobium, molybdenum, nickel-chromium, cobalt or alloys thereof, and combinations thereof.

In the present invention, the substrate may also include polyurethane, polyguanidine, polymethacrylate, polystyrene, substituted polystyrene, polysulfone, polysiloxane, polyamine, polyamide, polyacrylate, polymethacrylamide, polyacrylamide, polyazine, polyacrylonitrile, polyanhydride, polyalkene, polyester, poly(orthoester), polyether, polyetheretherketone (PEEK), polyolefin, polyurea, polyimide, poly(carbonate), polyketals, poly(ketones), polyphosphazines, polyfluorocarbons, poly(hydroxyalkanoates), Teflon, PTFE, natural and synthetic elastomers, polysaccharides, halogenated polymers, silicones, aldehyde crosslinking resins, epoxy resins, phenol resins, latex, Kevlar, Nomex, Dacron, nylon, or copolymers or blends thereof.

In the present invention, the medical device includes intravascular catheters, such as peripheral inserted central venous catheters (PICCs), central venous catheters (CVCs), or hemodialysis catheters, gastrostomy tubes, venous valves, punctal plugs, guide devices and implants. In addition, the substrate of the medical device may be an intravascular catheter formed of medical grade polyurethane or a material coated with medical grade polyurethane.

### Method of grafting quaternized trimethylamine on surface of substrate having hydroxyl group introduced therein

In another aspect, the present invention is directed to a method of grafting quaternized trimethylamine on the surface of a substrate, including introducing a hydroxyl group (-OH) into the surface of a substrate, further mixing the hydroxyl group-introduced substrate with an initiator, a reducing agent, and a trimethylamine-based compound, followed by reacting, to form a trimethylamine polymer layer on the surface of the substrate, and further mixing the substrate with hydrogen peroxide or meta-chloroperoxybenzoic acid, followed by reacting, to form a quaternized trimethylamine graft polymer layer on the surface of the substrate.

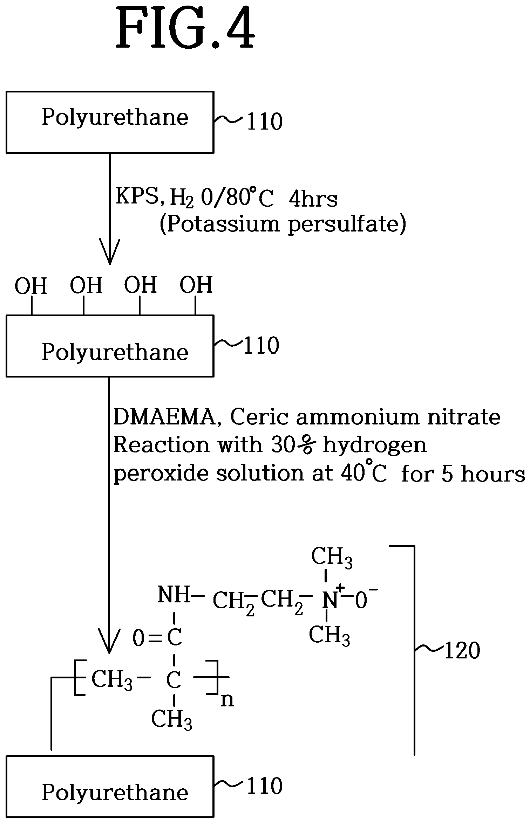

FIG. 4 is a schematic diagram illustrating a method of grafting an implantable medical device according to Example 3.

Specifically, FIG. 4 shows a grafting method using polyurethane.

Referring to FIG. 4, first, polyurethane 110, potassium persulfate (KPS) and distilled water were mixed and reacted to perform first surface modification of modifying a hydroxyl group (-OH) on the surface of the polyurethane 110.

In the subsequent step (second step), iron (II) gluconate (ferrous gluconate) as a reducing agent, ceric ammonium nitrate as an initiator, and a trimethylamine compound are added to the polyurethane 110 having the hydroxyl group introduced therein after the first surface modification to perform second surface modification to form a trimethylamine polymer layer on the surface of the polyurethane 110.

Hydrogen peroxide or meta-chloroperoxybenzoic acid is added to the polyurethane 110, on which the trimethylamine polymer layer is formed, and mixed to react the trimethylamine-based compound with hydrogen peroxide or meta-chloroperoxybenzoic acid to thereby perform third surface modification to form a quaternized trimethylamine graft polymer layer 120 on the surface of the polyurethane 110.

Specifically, as the first surface modification is performed, a hydroxyl group (-OH) is introduced into the surface of the polyurethane 110 to modify the surface of the polyurethane 110.

In addition, as the second surface modification is performed, the initiator penetrates into the polyurethane 110, the initiator induces initiation by the reducing agent to form radicals, and the formed radicals and the trimethylamine-based compound are polymerized to form a trimethylamine polymer layer.

That is, as the trimethylamine polymer layer is polymerized with radicals formed in the polyurethane 110, a strong physical bond with the polyurethane 110 is formed.

Meanwhile, through the first surface modification, a hydroxyl group (-OH group) formed on the surface of polyurethane 110 and the trimethylamine-based compound are polymerized to form a trimethylamine polymer layer. At this time, the hydroxyl group (-OH group) and the trimethylamine-based compound form a chemical bond.

That is, the trimethylamine polymer layer forms a strong physical bond in the polyurethane 110 and forms a chemical bond with the surface of the polyurethane 110, so that a trimethylamine polymer layer is formed as a polymer brush.

The method of grafting quaternized trimethylamine on the surface of a substrate having a hydroxyl group according to the present invention includes modifying the surface of the substrate with a hydroxyl group, without adding a compound having an isocyanate group, reacting a reducing agent and an initiator with a trimethylamine compound to form a trimethylamine polymer layer, and reacting trimethylamine with hydrogen peroxide or meta-chloroperoxybenzoic acid to graft quaternized trimethylamine on the substrate surface.

The description of the present invention may be applied as it is unless it contradicts the specific information described above.

### Method of grafting quaternized trimethylamine on surface of substrate having no isocyanate group introduced therein

In another aspect, the present invention is directed to a method of grafting quaternized trimethylamine on the surface of a substrate, including mixing a reaction solvent and an initiator with a substrate, followed by stirring, to modify the surface of the substrate, further mixing the surface-modified substrate with a reducing agent and a trimethylamine-based compound, followed by reacting, to form a trimethylamine polymer layer on the surface of the substrate, and further mixing the substrate with hydrogen peroxide or meta-chloroperoxybenzoic acid, followed by reacting, to form a quaternized trimethylamine graft polymer layer on the surface of the substrate.

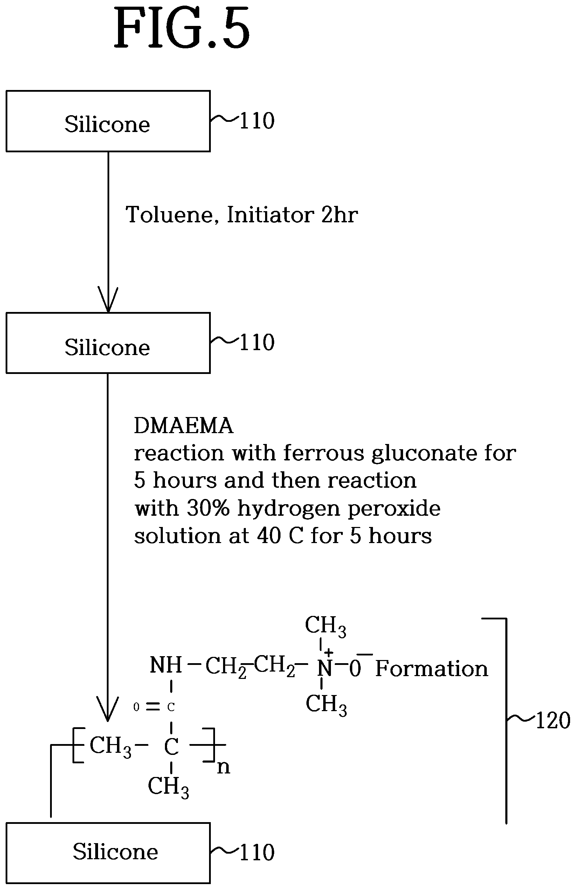

FIG. 5 is a schematic diagram illustrating a method of grafting an implantable medical device according to Example 3.

Specifically, FIG. 5 shows a grafting method using silicone 110.

Referring to FIG. 5, first, silicone 110, a solvent and an initiator are mixed and reacted to perform first surface modification.

In the subsequent step (second step), iron (II) gluconate (ferrous gluconate) as a reducing agent and a trimethylamine-based compound are added to the silicone 110 after the first surface modification to perform second surface modification to form a trimethylamine polymer layer on the surface of the silicone 110.

As the last step (the third step), hydrogen peroxide or meta-chloroperoxybenzoic acid is added to the silicone 110, and mixed to react the trimethylamine-based compound with hydrogen peroxide or meta-chloroperoxybenzoic acid to thereby perform third surface modification to form a quaternized trimethylamine graft polymer layer 120 on the surface of the silicone 110.

Specifically, as the surface modification is performed, the initiator penetrates into the polyurethane 110, the initiator induces initiation by the reducing agent to form radicals, and the formed radicals and the trimethylamine-based compound are polymerized to form a trimethylamine polymer layer.

The method of grafting quaternized trimethylamine on the surface of the substrate having no isocyanate group introduced therein according to the present invention includes modifying the surface of the substrate by reaction with an initiator, without adding a compound having an isocyanate group, reacting the surface of the substrate with a reducing agent and a trimethylamine compound to form a trimethylamine polymer layer, and reacting trimethylamine with hydrogen peroxide or meta-chloroperoxybenzoic acid to graft quaternized trimethylamine on the substrate surface.

The description of the present invention may be applied as it is unless it contradicts the specific information described above.

### [Mode for Invention]

Hereinafter, embodiments of the present invention will be described in detail with reference to the accompanying drawings so that the present invention can be implemented by those skilled in the art. However, the present invention may be embodied in many different forms and is not limited to the embodiments described herein.

### <Experimental Example 1> Preparation of polyurethane tube (catheter) and silicone tube (catheter)

### Example 1. Preparation of surface-treated polyurethane tube (catheter) by grafting

A polyurethane tube (catheter) with an outer diameter of 7FR and a length of 15 cm, acetonitrile, 5% by weight of isocyanatoethyl acrylate (ICEA), 0.03M dibutyltin dilaurate (DBTBL) and 1% by weight of polyether poly-t-butylperoxy carbonate (JWEB50) were mixed and stirred at 40°C for 2 hours.

Then, the pretreated polyurethane tube (catheter), 5 mM ferrous gluconate and 10% dimethylamino propyl methacrylamide (DMAPMA) were reacted in distilled water at 40°C for 10 hours to graft dimethylamino propyl methacrylamide on the polyurethane tube (catheter).

Then, the polyurethane tube (catheter) grafted with dimethylamino propyl methacrylamide was immersed in 200 mL of a hydrogen peroxide (30%) solution and reacted at 40°C to finally obtain a polyurethane tube (catheter) surface-modified with quaternized dimethylamino propyl methacrylamide (see FIG. 2).

### Example 2. Preparation of surface-treated polyurethane tube (catheter) by grafting

A polyurethane tube (catheter) with an outer diameter of 7FR and a length of 15 cm, 0.05% by weight of a bismuth carboxylate catalyst, 5% by weight of isocyanatoethyl acrylate (ICEA), and 1% by weight of polyether poly-t-butylperoxy carbonate (JWEB50) were mixed in toluene, stirred at 40°C for 2 hours and then was dried.

Then, the dried polyurethane tube (catheter) was reacted in distilled water at 40°C for 5 hours with 5 mM ferrous gluconate and 0.3 mol of dimethylamino propyl acrylamide (DMAPA), to graft the polyurethane tube (catheter) with dimethylamino propyl acrylamide.

Then, the polyurethane tube (catheter) grafted with dimethylamino propyl acrylamide was immersed in 200 mL of a hydrogen peroxide (30%) solution and reacted at 40°C to finally obtain a polyurethane tube (catheter) surface-modified with quaternized dimethylamino propyl acrylamide (FIG. 3).

### Example 3. Preparation of surface-treated polyurethane tube (catheter) by grafting

A polyurethane tube (catheter) having an outer diameter of 7 FR and a length of 15 cm was added to a 10% potassium persulfate solution (distilled water: 50 mL, potassium persulfate: 0.5 g) and reacted at 80°C for 4 hours, to introduce a hydroxyl group (-OH group) into the surface of the polyurethane tube (catheter).

The polyurethane tube (catheter) surface-modified with a hydroxyl group was washed with distilled water and dried at 40°C. Then, the dried polyurethane tube (catheter) was reacted in distilled water overnight.

Then, the reacted polyurethane tube (catheter) was immersed in an ethanol solution containing 1 wt% polyether poly-t-butylperoxy carbonate (JWEB50) and reacted for 2 hours.

The reacted polyurethane tube (catheter), a solution containing 50 mL distilled water and 5% dimethylamino ethyl methacrylate (DMAEMA), 0.05 g of ceric ammonium nitrate, 0.126 g of nitric acid and 5 mM ferrous gluconate were mixed and reacted for 5 hours to graft dimethylamino ethyl methacrylate on the polyurethane tube (catheter).

Then, the polyurethane tube (catheter) grafted with dimethylamino ethyl methacrylate was immersed in 200 mL of a hydrogen peroxide (30%) solution and reacted at 40°C to finally obtain a polyurethane tube (catheter) surface-modified with quaternized dimethylamino ethyl methacrylamide (FIG. 4).

### Example 4. Preparation of surface-treated silicone tube (catheter) by grafting

A silicone tube (catheter) was mixed with 1% by weight of polyether poly-t-butylperoxy carbonate (JWEB50) and stirred in toluene at 40°C for 2 hours and then was dried.

Then, the dried polyurethane tube (catheter), a solution containing 50 mL of distilled water and 5% dimethylamino ethyl methacrylate (DMAEMA), 5 mM ferrous gluconate were mixed and reacted for 5 hours to graft dimethylamino propyl methacrylamide on the silicone tube (catheter).

Then, the silicone tube (catheter) grafted with dimethylamino propyl methacrylamide was immersed in 200 mL of a hydrogen peroxide (30%) solution and reacted at 40°C to finally obtain a silicone tube (catheter) surface-modified with quaternized dimethylamino propyl methacrylamide (FIG. 5).

### Comparative Example 1. Preparation of surface-treated polyurethane tube (catheter) by grafting

50 g (0.294 mol) of dimethylamino propyl acrylamide (DMAPA) was stirred in an ice bath and 48.8 g of 30% hydrogen peroxide was slowly dropped therein for 30 minutes to induce reaction. Then, the reaction was performed at room temperature for 24 hours. The polyurethane tube (catheter) and 1 wt% polyether poly-t-butylperoxy carbonate (JWEB50) were stirred in ethanol and then the polyurethane tube was dried to graft the oxidized quaternized dimethylamino propyl acrylamide (N-oxide) on the polyurethane tube.

### <Experimental Example 2> Thrombus observation and grade measurement

### 1) Insertion of polyurethane tube (catheter) into beagles and evaluation of clot formation

The entire jugular vein surgical site of the beagle was depilated, disinfected with povidone and alcohol, and then covered with drapes. The left lateral jugular vein was exposed about 15 cm downward with respect to the thyroid cartilage of the beagle. The polyurethane tube (catheter) of Example 1 was inserted 10 ± 1 cm downward using the jugular vein parallel to the thyroid cartilage as the starting point. The distal end of the polyurethane tube (catheter) of Example 1, which was not inserted, was fixed to the surrounding cervical tissue by taping. The anesthesia was maintained for 4.0 ± 0.5 hours, a 10 ± 1 cm jugular vein including the site where the polyurethane tube (catheter) of Example 1 was inserted was extracted under cardiac anesthesia with a concentration of 5% or more of isoflurane. Anesthesia was further performed with 2.5 mg/kg of Zoletil and 1 mg/kg of xylazine by intramuscular injection and then was performed by intravenous injection of a large amount of KCl.

One side of the excised blood vessel was cut long, and the inside of the blood vessel was fully spread and fixed with a fixing pin. The blood vessel was imaged, the formation of clots was evaluated by scoring and the weight of the clots was measured using a precision electronic balance.

### 2) Clot formation evaluation result

The jugular vein was exposed in beagles, the polyurethane tube (catheter) of Example 1 was applied thereto for 3 hours, 1 day, 7 days, 14 days, 21 days, and 30 days, whether or not clots were formed inside the blood vessel and around the polyurethane tube (catheter) of Example 1 was observed, the weight of the clots was measured, and the degree of clot formation was evaluated by scoring.

**[Table 1]**

| Clot formation and grade criteria | |
|---|---|
| Grade | Formation of clots |
| Grade 0 | No clot was observed |
| Grade 1 | Small clots were observed in one site |
| Grade 2 | Small clots were observed in multiple sites |
| Grade 3 | Clots with less than 1/2 of catheter length were observed |
| Grade 4 | Clots with more than 1/2 of catheter length were observed |
| Grade 5 | Clogging of blood vessels was observed due to excessive blood clots |

### <Experimental Example 3> Blood coagulation and platelet test

Before the experiment, blood was collected in a 3.2% Sod. citrate tube from the cephalic vein of the test subjects, and PT (prothrombin time) and aPTT (activated partial thromboplastin time) tests relating to blood coagulation were performed. Whole blood anticoagulated with sodium citrate and blood at a ratio of 1:9 was measured using a coagulation analyzer (Coag Dx, IDEXX, USA). In addition, blood was collected in an EDTA tube and platelet tests were performed using an automatic hemacytometer (ADVIA 2120i, SIEMENS Healthineers, USA).
* Beagle blood normal range: PT = 11 to 17 (sec) / aPTT = 72 to 102 (sec) / Platelet = 143.3 to 400 (× 10³/µl)

Table 2 shows platelets, PTs, and aPTT measured 3 hours, 1 day, 7 days, 14 days, 21 days, and 30 days after insertion, as compared with before application of the polyurethane tube (catheter) of Example 1 according to the method described in Experimental Example 2 in beagles.

In the beagles, to which the polyurethane tube (catheter) of Example 1 was applied according to the method described in Experimental Example 2, the platelet value was changed from 218 (× 10³/µl) before insertion of the polyurethane tube (catheter) of Example 1, to 201, 197, 161, 163, 181, and 166, 3 hours, 1 day, 7 days, 14 days, 21 days, and 30 days, respectively, after insertion. The PT value was changed from 17 (sec) as the reference point to 17, 15, 14, 17, 14, and 14 at 3 hours, 1 day, 7 days, 14 days, 21 days, and 30 days after insertion. In addition, the aPTT value was changed from 82 (sec) to 95, 94, 94, 89, 86, and 83, respectively, at 3 hours, 1 day, 7 days, 14 days, 21 days, and 30 days after insertion. It was found that all values of platelets, PTs, and aPTTs measured in the beagles, to which the polyurethane tube (catheter) of Example 1 was applied, were within the normal range.

**[Table 2]**

| Coagulation | Pre | Post OP 3h | Post OP 1d | Post OP 7d | Post OP 14d | Post OP 21d | Post OP 30d |
|---|---|---|---|---|---|---|---|
| Platelet(× 10³/µl | 218 | 201 | 197 | 161 | 163 | 181 | 166 |
| PT(sec) | 14 | 17 | 15 | 14 | 17 | 14 | 14 |
| aPTT(sec) | 82 | 95 | 94 | 94 | 89 | 86 | 83 |

(In Table 2, Pre means before insertion of the polyurethane tube (catheter) of Example 1, and Post OD means after insertion of the polyurethane tube (catheter) of Example 1. The same meaning is used in Tables 3 and 4 below) .

### <Experimental Example 4> Weight measurement of beagles

The polyurethane tube (catheter) of Example 1 was applied according to the method described in Experimental Example 2 and the weight of the beagle was measured. Table 3 shows the result of comparison in the weight of the beagle, 3 hours, 1 day, 7 days, 14 days, 21 days, and 30 days after insertion, as compared with before application of the polyurethane tube (catheter) of Example 1.

**[Table 3]**

| | | | | | | | |
|---|---|---|---|---|---|---|---|
| Body weight gain | Pre | Post OP 3h | Post OP 1d | Post OP 7d | Post OP 14d | Post OP 21d | Post OP 30d |
| Weight(kg) | 10.5 | Not measured | Not measured | 10.6 | 10.5 | 10.8 | 10.8 |

The result showed that there was little change in body weight before and after application of the polyurethane tube (catheter) of Example 1.

### <Experimental Example 5> Measurement of blood inflammation

In order to compare the degree of blood inflammation in beagles before and after using the polyurethane tube (catheter) of Example 1 according to the method described in Experimental Example 2, the number of white blood cells (WBC), C-reactive protein (CRP) concentration and erythrocyte sedimentation rate (ESR) were measured. Table 4 shows the result of comparison in WBC count, CRP concentration, and ESR, 3 hours, 1 day, 7 days, 14 days, 21 days, and 30 days after insertion in comparison with before application of the polyurethane tube (catheter) of Example 1 to beagles.

The result showed that the number of WBC temporarily increased at the beginning (3 hours, 1 day) of application of the polyurethane tube (catheter) of Example 1, but gradually decreased over time. In addition, the CRP concentration also increased after the application of the polyurethane tube (catheter) of Example 1 at the beginning (3 hours, 1 day), but decreased over time. ESR also increased 14 and 30 days after application, but the others are the same as before application.

**[Table 4]**

| Inflammation | Pre | Post OP 3h | Post OP 1d | Post OP 7d | Post OP 14d | Post OP 21d | Post OP 30d |
|---|---|---|---|---|---|---|---|
| WBC(× 10³/µl) | 9.62 | 11.03 | 10.77 | 8.65 | 6.83 | 9.80 | 6.43 |
| CRP(mg/L) | 1.97 | 5.20 | 6.25 | 2.45 | 3.64 | 2.32 | 3.68 |
| ESR(mm) | 0.5 | 0.5 | 0.5 | 0.5 | 10 | 0.5 | 1.0 |

### <Experimental Example 6> Infrared spectroscopy (FT-IR) and H-NMR analysis

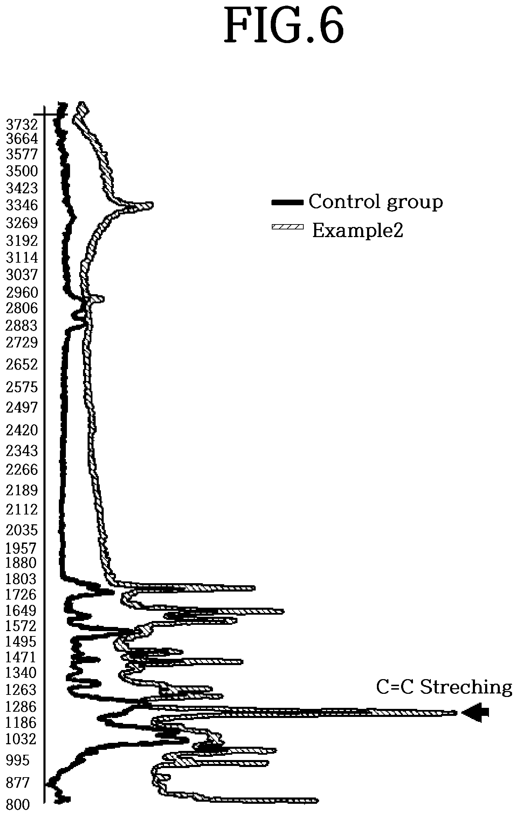

FIG. 6 shows the result of infrared spectroscopy analysis after an initiator and isocyanatoethyl acrylate (ICEA) were added to a polyurethane tube.

Referring to FIG. 6, -NH on the surface of the polyurethane tube (catheter) and -NCO group of ICEA were bonded in Example 2, which indicates that a C=C double bond was formed. Based thereon, it was predicted that the C=C group formed on the surface of the polyurethane 110 and trimethylamine could be polymerized to form a graft polymer.

Meanwhile, since, in Examples 1 to 4, the formation of quaternized trimethylamine by reaction with hydrogen peroxide cannot be measured by ¹H-NMR, after the trimethylamine-based compound was grafted on the substrate, in Comparative Example 1, whether or not quaternized trimethylamine was formed was indirectly determined before grafting on the substrate surface.

For this purpose, in Comparative Example 1, whether or not trimethylamine reacts with hydrogen peroxide to form quaternized trimethylamine before forming the graft polymer layer on the surface of the substrate was determined by ¹H-NMR analysis.

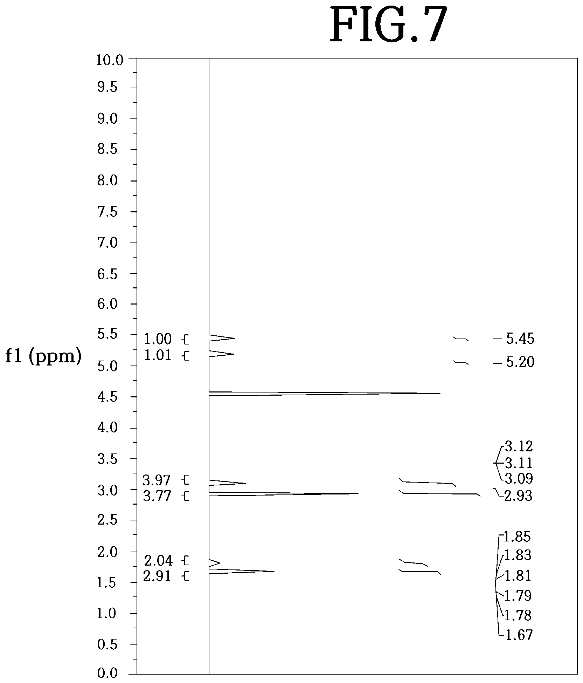

Referring to FIG. 7, it can be seen from ¹H-NMR analysis that DMAPA reacted with hydrogen peroxide to form quaternized dimethylamino propyl acrylamide.

### <Experimental Example 7> Surface characterization of graft tube (Water contact angle)

When hydration in which water molecules attach to the surface of the polyurethane tube occurs, a hydration layer is formed on the surface of the polyurethane tube to inhibit the adsorption of plasma proteins, serum, blood cells, platelets, and the like, and to inhibit the formation of biofilms of bacteria. Therefore, a water contact angle test was primarily conducted to determine the scale of the hydration phenomenon.

Specifically, about 0.2 mL of distilled water (DW) was injected into a syringe with a needle and fixed to a contact angle meter. A non-surface-treated polyurethane rod (control), surface-treated polyurethane rods (Examples 2 and 3), and a silicone rod (Example 4) were fixed to the contact angle meter and then the syringe was placed vertically. After focusing and adjusting the baseline, the experiment was conducted by inputting 0.75 ul, which is the amount required for one experiment. When the water droplet contacted the sample, the needle was raised and the contact angle was measured.

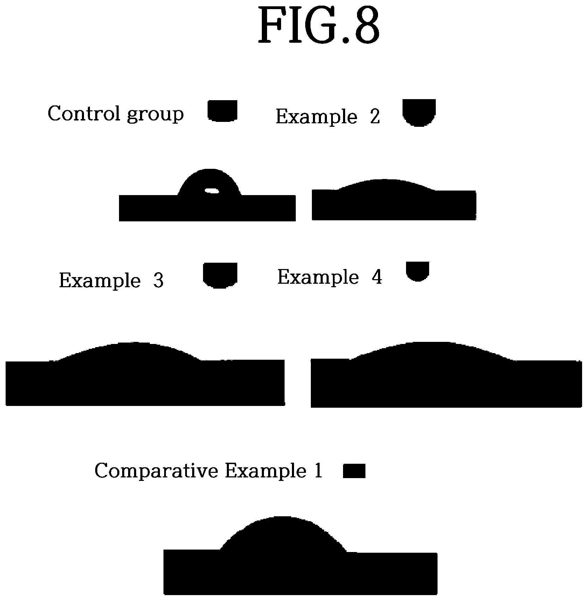

FIG. 8 shows the results of measurement of the contact angle of the surface of the substrate of Examples, Comparative Example and Control group, on which the graft polymer layer is formed, and Table 5 summarizes the results thereof.

Referring to FIG. 8 and Table 5, the contact angle of the control (non-surface-treated polyurethane tube) is 78.50°, the contact angle of the polyurethane tube (control) of Comparative Example 1 is 53.81°, and the surface contact angles of Examples 2 to 4, wherein the graft polymer layers are formed, were low, specifically, 23.18°, 27.78° and 23.18°, respectively.

This means that, as the graft polymer layer is formed on the surfaces of the polyurethane tube and the silicone tube, the surfaces of the polyurethane tube and the silicone tube are modified to be hydrophilic and thus the contact angles decrease. ?????

**[Table 5]**

| Contact angle | Control group | Example 2 | Example 3 | Example 4 | Comparative Example 1 |
|---|---|---|---|---|---|
| Unit: ° | 78.50° | 23.18° | 27.78° | 23.18° | 53.81° |

### <Experimental Example 8> Graft tube protein adsorption resistance effect - Fibrinogen adsorption experiment

The samples of Examples 2 to 4 were used as the graft tubes, quaternized dimethylamino propyl acrylamide was first formed by oxidation, and the non-surface-treated polyurethane tube (control), and a polyurethane tube (Comparative Example 1) was also used. The sample was subjected to protein adsorption experiments in the same manner as above.

The sample was cut into six specimens with a size of 1 cm × 1 cm. The prepared specimens were added to each well of a 24-well plate. 1 mL of PBS was added to the specimens and hydrated for 2 hours.

The PBS solution was removed from each well by suction, 1 ml of a fibrinogen solution (0.1 mg/ml of fibrinogen, Sigma-Aldrich)) was added thereto and allowed to stand at room temperature for 10 minutes.

Then, the fibrinogen solution was removed and the sample was repeatedly washed 4 to 5 times with the PBS solution. 1 ml of 2% by weight of a sodium dodecyl sulfate (SDS) solution was added to each well and stirred at room temperature at about 100 rpm for about 2 hours. The absorbance of the reactant was measured at a wavelength of 562 nm using a UV detector, the protein was assayed from the measured absorbance, and the results are shown in Table 6.

Referring to Table 6, the polyurethane rod of Example 2, on which the graft polymer layer was formed, was 0.0431 µg/cm², the polyurethane rod of Example 3 was 0.0459 µg/cm², and the silicone rod of Example 4 was 0.04989 µg/cm², whereas the polyurethane rod of control group was 0.1734 µg/cm² and the polyurethane rod of Comparative Example 1 was 0.1358 µg/cm².

That is, it was found that the amount of fibrinogen adsorbed was reduced by approximately 67 to 75% in the polyurethane rods of Examples 2 and 3 and the silicon rods in Example 4, compared to Control group and Comparative Example 1.

**[Table 6]**

| Amount of fibrinogen adsorbed | Control group | Example 2 | Example 3 | Example 4 | Comparative Example 1 |
|---|---|---|---|---|---|
| Unit: ug/cm² | 0.1734 | 0.0431 | 0.0459 | 0.04989 | 0.1358 |

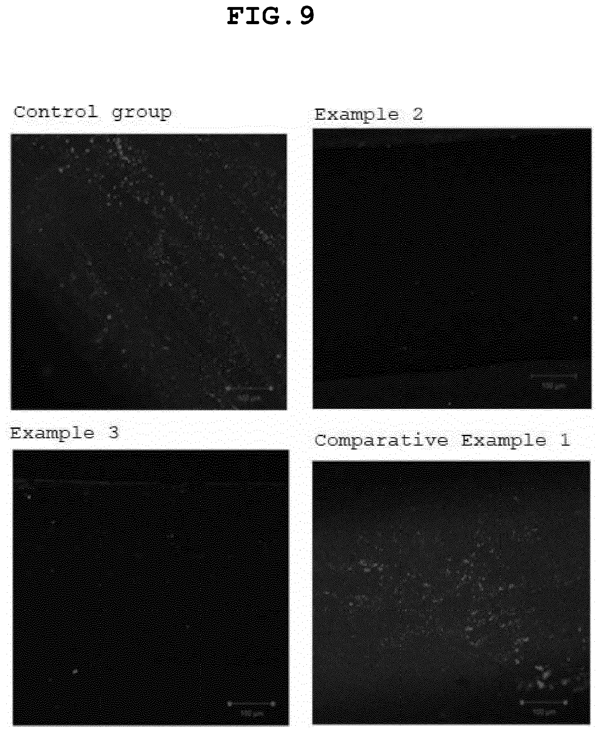

In addition, FIG. 9 is an image showing the adsorption amount of fibrinogen of Example, Comparative Example, and Control group. As can be seen from FIG. 9, fibrinogen is adsorbed on the surface of polyurethane in Comparative Example 1 and Control group. In contrast, it can be seen that the amount of fibrinogen observed in the polyurethane of Examples 2 and 3 and the silicone of Example 4 having the graft polymer layer formed thereon was remarkably reduced, compared to Comparative Example 1 and Control group.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A method of grafting quaternized trimethylamine on a surface of a substrate, comprising:
mixing a reaction solvent, a catalyst, an initiator, and a compound having an isocyanate group with a substrate, followed by stirring, to modify the surface of the substrate;
further mixing the surface-modified substrate with a reducing agent and a trimethylamine-based compound, followed by reacting, to form a trimethylamine polymer layer on the surface of the substrate; and
further mixing the substrate with hydrogen peroxide or meta-chloroperoxybenzoic acid, followed by reacting, to form a quaternized trimethylamine graft polymer layer on the surface of the substrate.

2. The method according to claim 1, wherein a water contact angle of the surface of the substrate modified with quaternized trimethylamine is 60° or less, and an amount of fibrinogen adsorbed on the surface thereof is 1 µg/cm² or less.

3. The method according to claim 1, wherein the catalyst is selected from the group consisting of dibutyltin dilaurate, triethylamine, diethylenetriamine, bismuth carboxylate, and zirconium chelate.

4. The method according to claim 1, wherein the initiator is selected from the group consisting of an ultraviolet initiator, a thermal initiator, and a redox initiator.

5. The method according to claim 1, wherein the initiator is selected from the group consisting of 2,2-bis(4,4-di(t-butylperoxy)cyclohexyl)propane, tri(t-butylperoxy)triazine, tri(t-butylperoxy)trimellitate, polyether poly-t-butyl peroxycarbonate, t-butyl peroxy methyl fumarate, and t-butyl peroxy ethyl fumarate.

6. The method according to claim 1, wherein the compound having an isocyanate group is selected from the group consisting of 2-isocyanatoethyl methacrylate (IEM), 2-isocyanatoethyl acrylate (ICEA), isophorone diisocyanate, and hexamethylene diisocyanate.

7. The method according to claim 1, wherein the substrate comprises a metal, ceramic, glass, a synthetic polymer, biological tissue, woven fiber, non-woven fabric, a semi-metal, or silicone.

8. The method according to claim 1, wherein the trimethylamine compound comprises at least one selected from the group consisting of dimethylamino propyl methacrylamide, dimethylamino propyl acrylamide, and dimethylamino ethyl methacrylate.

9. The method according to claim 1, wherein the reducing agent is selected from the group consisting of metal salts, sulfur oxyacids, alcohols, hydroxy acids, thiols, ketones, amine aldehydes, and amides.

10. The method according to claim 9, wherein the metal salt is a salt of Fe(II), Cu(II), Cr(II), V(II), Ti (III) or Ag(I) .

11. The method according to claim 1, wherein the quaternized trimethylamine is quaternized dimethylamino propyl methacrylamide, quaternized dimethylamino propyl acrylamide, or quaternized dimethylamino ethyl methacrylate.

12. A method of grafting quaternized trimethylamine on a surface of a substrate, comprising:
introducing a hydroxyl group (-OH) into the surface of the substrate;
further mixing the hydroxyl group-introduced substrate with an initiator, a reducing agent, and a trimethylamine-based compound, followed by reacting, to form a trimethylamine polymer layer on the surface of the substrate; and
further mixing the substrate with hydrogen peroxide or meta-chloroperoxybenzoic acid, followed by reacting, to form a quaternized trimethylamine graft polymer layer on the surface of the substrate.

13. A method of grafting quaternized trimethylamine on a surface of a substrate, comprising:
mixing a reaction solvent and an initiator with a substrate, followed by stirring, to modify the surface of the substrate;
further mixing the surface-modified substrate with a reducing agent and a trimethylamine-based compound, followed by reacting, to form a trimethylamine polymer layer on the surface of the substrate; and
further mixing the substrate with hydrogen peroxide or meta-chloroperoxybenzoic acid, followed by reacting, to form a quaternized trimethylamine graft polymer layer on the surface of the substrate.
